# EUROPÄISCHE PATENTSCHRIFT

(11) **EP 0 560 368 B1**
(45) Veröffentlichungstag und Bekanntmachung des Hinweises auf die Patenterteilung: **11.02.1998**
(21) Anmeldenummer: 93103965.5
(22) Anmeldetag: 11.03.1993
(51) Int. Cl.: A61M 1/16

(54) **Verfahren zum On-Line Spülen und Befüllen eines extrakorporalen Blutkreislaufes von Dialysemaschinen**
Process for on-line cleaning and filling of an extra corporeal blood circuit of a dialysis apparatus
Procédé de nettoyage et remplissage en ligne d'un circuit extracorporel sanguin d'une machine de dialyse

(30) Priorität: 13.03.1992 DE 4208274
(43) Veröffentlichungstag der Anmeldung: 15.09.1993
(60) Teilanmeldung: 96103982.3
(73) Patentinhaber: MEDICAL SUPPORT GMBH, 97076 Würzburg (DE)
(72) Erfinder: Eigendorf, Hans-Günther Dr., O-1242 Bad Saarow-Pieskow (DE)
(74) Vertreter: Flosdorff, Jürgen, Dr.

(56) Entgegenhaltungen:
- EP-A- 0 199 518
- EP-A- 0 234 001
- DE-C- 3 936 785
- FR-A- 2 175 945
- FR-A- 2 296 432
- FR-A- 2 368 963
- US-A- 4 331 540
- US-A- 4 784 771

## Beschreibung

Die Erfindung betrifft ein Verfahren zum Spülen und Befüllen eines extrakorporalen Blutkreislaufs von Dialysemaschinen, insbesondere von Blutschlauchleitungen und Dialysatoren.

Die Erfindung betrifft ferner die Anwendung des Verfahrens in Verbindung mit einer Hämodiafiltration.

Als arterielle und venöse Blutschlauchleitung eines extrakorporalen Blutkreislaufs werden heutzutage üblicherweise steril verpackte Einmalartikel verwendet, die vor einer Behandlung gespült, entlüftet und befüllt werden müssen. Dies geschieht bisher mittels physiologischer Kochsalzlösungen, die mittels der maschineneigenen Blutpumpe durch das Schlauchsystem und den Dialysator gefördert werden, um den extrakorporalen Blutkreislauf von Verunreinigungen freizuspülen und unter vollständiger Entlüftung zu befüllen.

Die Verwendung der Kochsalzspülflüssigkeit hat den Nachteil, daß sie mit erheblichen Kosten verbunden ist.

Aus der FR-A-2175945 ist ein Verfahren bekannt, bei dem von einer Dialysemaschine erzeugte Dialysierflüssigkeit durch einen extrakorporalen Blutkreislauf gefördert wird. In dieser Druckschrift wird vorgeschlagen, zur Spülung des extrakorporalen Blutkreislaufs nach Beendigung einer Dialyse die beiden Schläuche des extrakorporalen Blutkreislaufs mit den Leitungen des Dialysierflüssigkeitskreislaufs zu verbinden.

Der vorliegenden Erfindung liegt die Aufgabe zugrunde, ein Verfahren zur Spülung, Entlüftung und Befüllung eines extrakorporalen Blutkreislaufs von Dialysemaschinen anzugeben, das mit geringeren Kosten verbunden ist. Außerdem soll ein Anordnung angegeben werden, mit der dieses Verfahren ausführbar ist.

Diese Aufgabe wird erfindungsgemäß durch die im Kennzeichen des Anspruchs 1 angegebenen Merkmale gelöst.Vorteilhafte Weiterbildungen der Erfindung sind in den abhängigen Ansprüchen gekennzeichnet.

Nach dem erfindungsgemäßen Verfahren wird die von der Dialysemaschine erzeugte bzw. aufbereitete Dialysierflüssigkeit zum Zwecke des Spülens, Entlüftens und Befüllens durch den bzw. in den extrakorporalen Blutkreislauf gefördert. Hierdurch entfällt das bisherige Erfordernis, eine in einem Beutel abgefüllte Kochsalzlösung zu diesem Zweck bereit zu stellen, der zudem von einer Bedienungsperson an die arterielle Blutschlauchleitung anzuschließen war. Es entfällt damit ferner das Erfordernis, zu dem genannten Zweck Kochsalzlösungen in größeren Mengen bereit zu halten und die geleerten Beutel zu entsorgen.

Gemäß der Erfindung wird die Dialysierflüssigkeit blutseitig des Dialysators abgenommen, wobei sie beim Durchtritt durch die Membran filtriert wird. Hiermit ist die Keimfreiheit der Spül- und Befüllflüssigkeit zuverlässig gewährleistet.

Nach einem weiteren Vorschlag der Erfindung kann die dem Dialysator entnommene Dialysierflüssigkeit von der maschineneigenen Blutpumpe durch den extrakorporalen Blutkreislauf gepumpt werden, was nachfolgend in näheren Einzelheiten erläutert wird. Bei dieser Ausgestaltung der Erfindung wird die Saugseite der arteriellen Blutschlauchleitung vorzugsweise mittels eines Einmalartikels an dem Dialysator konnektiert, vorzugsweise an der Stelle, an der bei der Dialysebehandlung die venöse Blutschlauchleitung angeschlossen wird. Das Blutpumpensegment der arteriellen Blutschlauchleitung wird in die maschineneigene Blutpumpe, vorzugsweise eine Rollerpumpe, eingelegt, und das andere, freie Ende der arteriellen Blutschlauchleitung wird vorzugsweise über einen weiteren Einmalartikel mit dem Einlauf der venösen Blutschlauchleitung konnektiert. Die venöse Blutschlauchleitung durchläuft auf übliche Weise einen Luftdetektor. Das andere Ende der venösen Blutschlauchleitung wird stromabwärts des Dialysators an den Dialsysierflüssigkeitskreislauf angeschlossen, vorzugsweise mittels eines weiteren Einmalartikels an ein Probeentnahmeventil dieses Dialysierflüssigkeitskreislaufs.

Wenn die Blutpumpe in Betrieb gesetzt wird, wird Dialysierflüssigkeit über die semipermeable Membran des Dialysators in die Blutschlauchleitungen gezogen. Dabei gelangt nur sterile und keimfreie Spülflüssigkeit in den extrakorporalen Blutkreislauf. Die Spülflüssigkeit und die in dem extrakorporalen Kreislauf zuvor befindliche Luft werden stromabwärts des Dialysators in den Dialysierflüssigkeitskreislauf der Dialysemaschine eingeführt, womit bilanz- und sonstige maschinenseitigen Störungen vermieden sind.

Nach einem alternativen Vorschlag der Erfindung wird die Dialysierflüssigkeit zum Zwecke der Spülung, Entlüftung und Befüllung des extrakorporalen Blutkreislaufs nicht mittels der maschineneigenen Blutpumpe, sondern von einer Pumpe des Dialysierflüssigkeitskreislaufs durch die bzw. in die Blutschlauchleitungen gepumpt. Hierbei kann dieser Vorgang durch das maschinenspezifische Füllprogramm hervorgerufen werden.

Im einzelnen ist bei dieser bevorzugten Ausführungsform des erfindungsgemäßen Verfahrens vorgesehen, daß die arterielle und die venöse Blutschlauchleitung in der Weise an dem Dialysator konnektiert werden, wie dies bei einer Dialysebehandlung der Fall ist. In dem Dialysierflüssigkeitskreislauf ist stromabwärts des Dialysators ein Dreiwege-Ventil mit 2/2 Wegen angeordnet, mit dem der Dialysierflüssigkeitskreislauf ganz oder teilweise verschließbar ist bzw. unterbrochen werden kann. Das dritte Ventil dieses Dreiwege-Hahns, der vorzugsweise mechanisch einfach zu verstellen ist, liegt nicht im Dialysierflüssigkeitskreislauf und kann eine zur Atmosphäre hin offene Verbindung schaffen.

Zum Spülen und Befüllen des extrakorporlane Blutkreislaufs wird der Dreiwege-Hahn so geschaltet, daß der Dialysierflüssigkeitskreislauf zur Atmosphäre hin geöffnet ist, während die von dem Dialysator wegführende Leitung ganz oder teilweise verschlossen ist. Es wird betont, daß eine Zwischenstellung des Dreiwegehahns eingestellt werden kann, in der noch ein Teil der Dialysierflüssigkeit über die Ventilanordnung abfließen kann, was weiter unten noch erläutert wird.

Wenn der Dialysierflüssigkeitskreislauf durch entsprechende Schaltung des Dreiwegehahns zur Atmosphäre hin geöffnet ist, kann die maschineneigene Sensorik die Luft erkennen, die über den zugeordneten Anschluß ins Maschineninnere gelangt, und das maschinenspezifische Füllprogramm auslösen. Die dabei von der Maschine kommende Dialysierflüssigkeit wird über den Dialysator in die beiden angeschlossenen Blutschlauchleitungen gedrückt, da der Dreiwegehahn den Dialysierflüssigkeitskreislauf ganz oder teilweise unterbrochen hat. Somit werden die Blutschlauchleitungen gespült, entlüftet und mit der Dialysierflüssigkeit befüllt.

Die patientenseitigen Anschlüsse werden nach einem weiteren Vorschlag der Erfindung beispielsweise an einen leeren Beutel konnektiert. In diesem Fall kann es sich empfehlen, den Dreiwegehahn auf eine Zwischenstellung zu schalten, um den Dialysierflüssigkeitskreislauf nur teilweise zu verschließen, damit der Beutel nicht mit einer zu großen Spülflüssigkeitsmenge gefüllt bzw. nicht mit zu großem Druck belastet wird, der ihn platzen lassen könnte.

Nach einem alternativen Vorschlag der Erfindung können die patientenseitigen Anschlüsse der beiden Blutschlauchleitungen an den dritten, nicht im Dialysierflüssigkeitskreislauf liegenden Anschluß des Dreiwegebahns konnektiert werden, und zwar vorzugsweise mittels eines geeigneten Einmalartikels. Eine weitere Möglichkeit besteht darin, daß die freien Enden der Blutschlauchleitungen so angeordnet werden, daß sie in ein offenes Gefäß einmünden.

Bei dieser letztgenannten Ausführungsform des erfindungsgemäßen Verfahrens bzw. der erfindungsgemäßen Anordnung wird das arterielle Pumpsegment nicht in die Blutpumpe eingelegt, da die Befüllung der arteriellen Blutschlauchleitung in umgekehrter Richtung erfolgt.

Ein Vorteil der vorstehend beschriebenen zweiten Variante besteht darin, daß dialysatorseitig keine sterilen Einmalartikel benötigt werden und daß die arterielle und die venöse Blutschlauchleitung wie gewohnt konnektiert werden und unverändert bleiben.

Die Spül- und Befüllvorgang muß nicht notwendigerweise durch das maschinenspezifische Füllprogramm ausgelöst werden, welches auf ins Maschineninnere gelangte Luft anspricht.

Eine dritte Variante des erfindungsgemäßen Verfahrens sieht vor, daß nach dem Konnektieren der arteriellen und venösen Blutschlauchleitung am Dialysator die Patientenanschlüsse dieser beiden Leitungen miteinander verbunden werden, um eine durchgehende Flüssigkeitsleitung zu bilden, daß der Strömungsweg zwischen dem venösen Blutanschluß des Dialysators und der zur Entlüftung vorzugsweise vorgesehenen venösen Tropfkammer gesperrt wird, daß die Blutpumpe entgegen ihrer normalen Förderrichtung in Betrieb gesetzt wird, und daß die aus dem Blutschlauchsystem verdrängte Luft und überschüssige Flüssigkeit durch eine mit dem oberen Teil der venösen Tropfkammer verbundene Leitung abgeleitet wird. Dabei kann vorgesehen sein, daß die verdrängte Luft und die überschüssige Flüssigkeit in das Dialysierflüssigkeitsystem des Dialysegerätes abgeführt werden.

Dieses erfindungsgemäße Verfahren kann dadurch weiter ausgestaltet werden, daß intermittierend die normalen Strömungsverhältnisse des extrakorporalen Blutkreislaufs hergestellt werden, d.h. Öffnen des Strömungsweges zwischen dem venösen Blutanschluß des Dialysators und der venösen Tropfkammer und Inbetriebsetzen der Blutpumpe in ihrer normalen Förderrichtung, wobei die mit dem oberen Teil der venösen Tropfkammer verbundene Leitung verschlossen ist.

Das Umschalten der Ventile und der Förderrichtung der Blutpumpe kann über ein automatisch ablaufendes Programm im Rahmen des Steuerungsprogramms des Dialysegerätes bewirkt werden.

Bei einer weiteren Variante des erfindungsgemäßen Verfahrens werden wie bei der dritten Ausführungsform der arterielle und der venöse Patientenanschluß der Blutschlauchleitungen zur Schaffung einer durchgehenden Flüssigkeitsleitung miteinander verbunden, und eine an den oberen Teil der venösen Tropfkammer angeschlossene Leitung wird mit einer Saugvorrichtung versehen bzw. verbunden. Hierbei kann als Saugvorrichtung das Entgasungssystem des Dialysegerätes verwendet werden, wobei alternativ eine Pumpe angeordnet sein kann, deren abfördernde Leitung mit dem Dialysierflüssigkeitssystem verbunden sein kann.

In weiterer Ausgestaltung des erfindungsgemäßen Verfahrens kann vorgesehen sein, daß in der Dialysatkammer des Dialysators in vorgegebenen Zeitabständen ein erhöhter Druck erzeugt wird, der den Druck in der Blutkammer des Dialysators übersteigt und eine Filtration von Dialysierflüssigkeit in das Blut bewirkt. Dieser nach dem Anschließen eines Patienten und dem Beginn der eigentlichen Behandlung erfolgende Vorgang dient dazu, die Dialysemembran entgegen der normalen Filtrationsrichtung periodisch freizuspülen, d. h. Ablagerungen, die sich auf der Blutseite der Membran angesammelt haben, zu entfernen.

Vorzugsweise wird bei diesem Verfahren ein volumenstarr wirkendes Dialysierflüssigkeitssystem verwendet, wodurch die Möglichkeit geschaffen ist, daß eine periodische Druckerhöhung und Druckabsenkung mit der Pumpe bewirkt wird, die abwechselnd zusätzliche Flüssigkeit in das Dialysierflüssigkeitssystem einführt bzw. dem Dialysierflüssigkeitssystem Flüssigkeit entzieht, was in näheren Einzelheiten weiter unten noch beschrieben wird.

Erfindungsgemäß kann dieses Verfahren in Verbindung mit einer Hämodiafiltration angewendet werden.

Nach einem zusätzlichen Vorschlag der Erfindung ist vorgesehen, daß ein Sterilfilter stromaufwärts des Dialysators in dem Dialysierflüssigkeitskreislauf angeordnet wird, um die Sicherheit in Bezug auf die erforderliche Keimfreiheit zusätzlich zu erhöhen.

Nach einem weiteren Gesichtspunkt der Erfindung wird vorgeschlagen, daß die als Spülflüssigkeit verwendete Dialysierflüssigkeit nach Beendigung einer Dialysebehandlung zur Freispülung des extrakorporalen Blutkreislaufes verwendet wird, wozu es zweckmäßig ist, diese Flüssigkeit beim Spülvorgang in einen Beutel abzuführen. Das Freispülen ist notwendig, um nach der Dialysebehandlung das Blut vollständig zum Patienten zurückzuführen, was bisher ebenfalls mit Hilfe einer Kochsalzlösung geschieht.

Ein weiterer erfindungsgemäßer Vorschlag geht dahin, die extrakorporalen Komponenten so zu reinigen, daß sie wiederverwendbar sind. Hierzu kann die Anordnung so getroffen werden, wie sie weiter oben im Zusammenhang mit der ersten Ausführungsform des erfindungsgemäßen Verfahrens beschrieben ist. Bei einem dialysemaschinenspezifischen Reinigungsprogramm wird durch die Förderung der Blutpumpe Reinigungsflüssigkeit über die semipermeable Membran des Dialysators in den extrakorporalen Kreislauf gezogen, wodurch dieser entsprechend gereinigt wird.

Selbstverständlich liegt es im Rahmen der Erfindung, die extrakorporalen Komponenten weiterhin als Einmalartikel einzusetzen.

Weitere Merkmale, Vorteile und Einzelheiten der Erfindung ergeben sich aus der nachfolgenden Beschreibung einiger bevorzugter Ausführungsformen sowie anhand der Zeichnung. Dabei zeigen auf rein schematische Weise:
- Fig. 1: eine erste Ausführungsform zur Ausführung des erfindungsgemäßen Verfahrens;
- Fig. 2: eine herkömmliche Anordnung;
- Fig. 3: eine zweite Ausführungsform zur Ausführung des erfindungsgemäßen Verfahrens;
- Fig. 4: eine dritte Ausführungsform zur Ausführung des erfindungsgemäßen Verfahrens;
- Fig. 5: eine vierte Ausführungsform zur Ausführung des erfindungsgemäßen Verfahrens und
- Fig. 6: eine fünfte Ausführungsform zur Ausführung des erfindungsgemäßen Verfahrens.

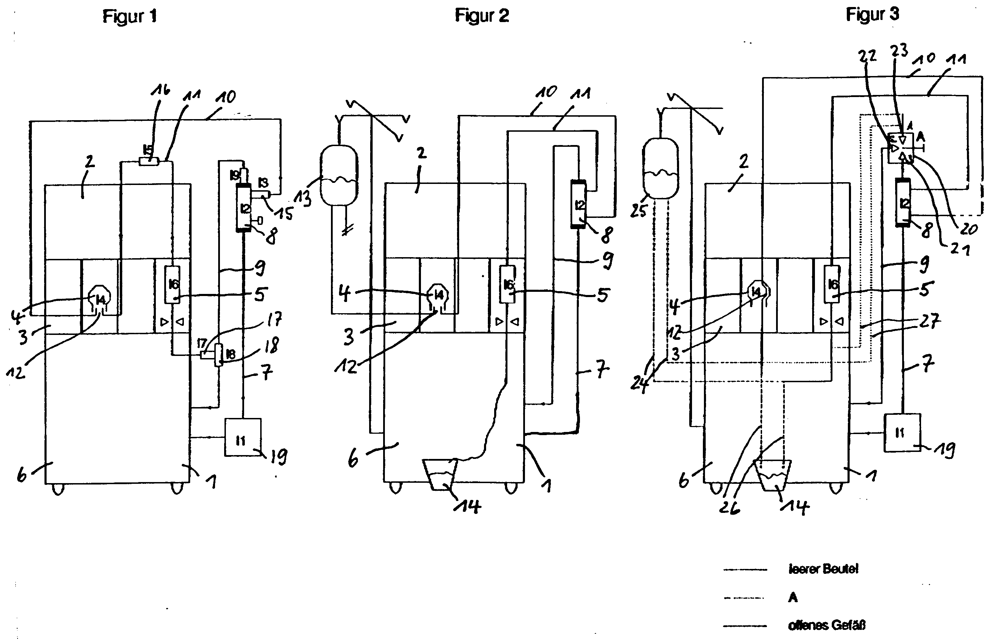

Es wird zunächst auf Fig. 2 Bezug genommen, um das bisher übliche Verfahren zum Spülen, Entlüften und Befüllen eines extrakorporalen Blutkreislaufs zu erläutern. Die Fig. zeigt eine eigentliche Dialysemaschine 1 mit der oberen Überwachungsebene 2, der mittleren Ebene 3, die eine Blutpumpe 4 und einen Luftdetektor 5 enthält, und mit einer unteren Ebene 6, die den Hydraulikteil aufweist.

Die Dialysemaschine 1 ist über eine Zuleitung 7 mit dem Dialysator 8 verbunden, von dem eine Rückleitung 9 zur Dialysemaschine 1 zurückführt, um einen Dialysierflüssigkeitskreislauf zu schließen.

Zum Zwecke einer Dialysebehandlung werden eine arterielle Blutschlauchleitung 10 und eine venöse Blutschlauchleitung 11 an den dargestellten Stellen an dem Dialysator 8 konnektiert. Ein Blutpumpensegment 12 der arteriellen Blutschlauchleitung 10 wird in die Blutpumpe 4 eingelegt, während die venöse Blutschlauchleitung 11 über den Luftdetektor 5 führt.

Zur Vorbereitung einer Dialysebehandlung müssen die Blutschlauchleitungen 10 und 11 freigespült werden und der extrakorporale Blutkreislauf muß entlüftet und befüllt werden. Hierzu ist es bisher üblich, das freie Ende der arteriellen Blutschlauchleitung 10 mit dem Ausgang eines eine Kochsalzlösung enthaltenden Beutels 13 zu verbinden. Die Blutpumpe 4 fördert die Kochsalzlösung durch die arterielle Blutschlauchleitung 10, den Dialysator 8, die venöse Blutschlauchleitung 11 und den Luftdetektor 5, um schließlich die Spülflüssigkeit in einen offenen Behälter 14 abzuführen.

Es wird nun auf Fig. 1 Bezug genommen, in der -ebenso wie in Fig. 3 bis 6- die Bauteile, die mit denjenigen gemäß Fig. 2 übereinstimmen, mit denselben Bezugszeichen gekennzeichnet sind. Auf ihre Wiederholung wird aus Gründen der Übersichtlichkeit verzichtet.

Bei dieser Ausführungsform der Erfindung wird die arterielle Blutschlauchleitung 10 mittels eines Einmalartikels 15 an der Stelle an dem Dialysator 8 konnektiert, an der gemäß Fig. 2 die venöse Blutschlauchleitung 11 angeschlossen ist. Das Blutpumpensegment 12 der arteriellen Blutschlauchleitung 10 ist mittels eines Einmalartikels 16 mit der venösen Blutschlauchleitung 11 verbunden. Die venöse Blutschlauchleitung 11 führt wiederum über den Luftdetektor 5 und ist mittels eines Einmalartikels 17 an ein Probeentnahmeventil 18 angeschlossen.

In der Leitung 7 des Dialysierflüssigkeitskreislaufs ist ein Sterilfilter 19 angeordnet, der die Sicherheit der Keimfreiheit der Spülflüssigkeit weiter erhöht.

Durch Förderung der Blutpumpe 4 gelangt Dialysierflüssigkeit aus dem Dialysierflüssigkeitskreislauf 7, 8, 9 in die arterielle Schlauchleitung 10 und die venöse Schlauchleitung 11, von wo sie in den Dialysierflüssigkeitskreislauf (bei 18) zurückgegeben wird, um bilanz- und sonstige maschinenseitigen Störungen zu vermeiden.

Bei der erfindungsgemäßen Ausführungsform gemäß Fig. 3 werden die arterielle Blutschlauchleitung 10 und die venöse Blutschlauchleitung 11 in der Weise an dem Dialysator 8 konnektiert, wie dies im Zusammenhang mit der Figur 2 beschrieben und bei einer Dialysebehandlung der Fall ist. Bei dieser Ausführungsform ist ein Dreiwegehahn 20 in der Leitung 9 angeordnet, dessen Anschlüsse 21 und 22 der Leitung 9 zugeordnet sind, während sein Anschluß 23 normalerweise zur Atmosphäre hin offen ist.

Während einer Dialysebehandlung besteht eine Verbindung von 21 nach 22, während -wie gesagt- Anschluß 23 zur Atmosphäre hin offen ist. Zum Spülen des extrakorporalen Blutkreislaufs wird die Ventilanordnung 20 umgeschaltet auf Durchgang 22, 23, wobei auch eine Zwischenstellung schaltbar ist, in der ein eingeschränkter Durchgang 21, 22 bestehen bleibt. Von der Dialysemaschine 1 kommende Dialysierflüssigkeit wird über den Dialysator 8 in die Blutschlauchleitungen 10, 11 gedrückt, wobei diese durchgespült werden. Die Spülflüssigkeit kann entweder über Leitungen 24 in einen Beutel 25, über Leitungen 26 in das offene Gefäß 14 abgeführt oder über Leitungen 27 dem Anschluß 23 des Dreiwegeventils 20 zugeführt wreden, von wo die Flüssigkeit in den Dialysierflüssigkeitskreislauf bzw. dessen Leitung 9 gelangt.

Bei dieser Ausführungsform ist das Blutpumpensegment nicht in die Blutpumpe 4 eingelegt.

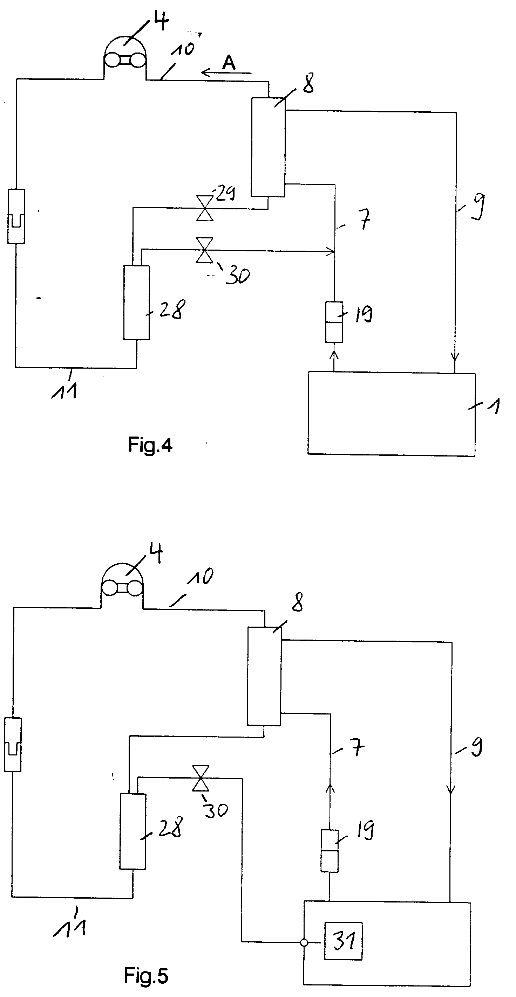

Ein weiteres Ausführungsbeispiel ist in Fig. 4 gezeigt. Beim Füll- und Spülvorgang sind die patientenseitigen Anschlüsse des areriellen und des venösen Blutschlauches 10, 11 miteinander verbunden. In die venöse Blutleitung 11 zwischen dem Dialysator 8 und der venösen Tropfkammer 28 ist ein Ventil 29 in Form einer Schlauchklemme eingefügt. Vom oberen Teil der venösen Tropfkammer 28 zweigt eine ebenfalls mit einem Ventil 30 ausgestattete Leitung ab, die mit einem Auffanggefäß oder, wie in Fig. 4 dargestellt, mit dem Dialysierflüssigkeitssystem verbunden ist.

Zum Zwecke des Füllens und Spülens des extrakorporalen Blutkreislaufes wird die Blutpumpe 4 entgegen ihrer normalen Förderrichtung (Richtungspfeil A) in Tätigkeit gesetzt (z.B. durch Ändern ihrer Drehrichtung), das Ventil 29 wird geschlossen und das Ventil 30 geöffnet.

Durch die Tätigkeit der Blutpumpe entsteht im Blutkompartiment des Dialysators 8 ein Unterdruck. Dadurch tritt filtrierte Dialysierflüssigkeit in das Blutkompartiment und das Blutschlauchsystem über, so daß dieses in kurzer Zeit gefüllt wird. Die abgepumpte Luft und nachfolgende Flüssigkeit werden über das geöffnete Ventil 30 in das Dialysierflüssigkeitssystem gefördert.

Dieses Verfahren kann zur Intensivierung des Spülvorgangs noch dadurch ergänzt werden, daß zwischenzeitlich mehrfach das Ventil 29 geöffnet wird (bei gleichzeitigem oder zeitlich versetztem Schließen des Ventils 30) und bei Bedarf zusätzlich die Blutpumpe in ihrer normalen Förderrichtung (entgegen dem Richtungspfeil A) arbeitet.

Die genannten Umschaltungen der Ventile 29, 30 und der Förderrichtung der Blutpumpe 4 können ohne weiteres über ein automatisch ablaufendes Programm im Rahmen des Steuerungsprogramms des Dialysegerätes bewirkt werden, so daß sich eine erhebliche Arbeitsvereinfachung erreichen läßt.

Fig. 5 zeigt ein weiteres Ausführungsbeispiel. Die patientenseitigen Anschlüsse der Blutschläuche sind zum Zwecke des Füllens und Spülens des extrakorporalen Blutkreislaufes miteinander verbunden. Eine vom oberen Teil der venösen Tropfkammer abzweigende Leitung steht mit einer Saugvorrichtung 31 in Verbindung, die dazu dient, im gesamten extrakorporalen Leitungssystem, einschließlich des Blutkompartiments des Dialysators 8, einen Unterdruck zu erzeugen, so daß filtrierte Dialysierflüssigkeit in das Blutkompartiment übertritt. Bei eingeschalteter Blutpumpe 4 zirkuliert diese Flüssigkeit dann durch den gesamten Kreislauf. Luft und überschüssige Flüssigkeit werden über die vom oberen Teil der venösen Tropfkammer abzweigende Leitung abgeführt.

Die Saugvorrichtung 31 kann eine externe Saugvorrichtung sein, z.B. unter Benutzung des in Krankenhäusern häufig verfügbaren Vakuum-Leitungsnetzes, ggf. mit einem zwischengeschalteten Flüssigkeitsabscheider, oder sie kann als Pumpe ausgebildet sein, wobei die austretende Flüssigkeit in einem Auffanggefäß gesammelt oder in das Dialysierflüssigkeitssystem abgeleitet werden kann. Eine besonders einfache und damit vorteilhafte Anordnung ergibt sich, wenn als Saugvorrichtung das Entgasungssystem des Dialysegerätes mitbenutzt wird. Dabei handelt es sich bei den meisten Dialysegeräten um eine Pumpe, die in einem Teilabschnitt der stromabwärts von ihr liegenden Fließstrecke einen starken Unterdruck erzeugt, um überschüssig gelöstes Gas zur Ausscheidung zu bringen. Die Saugtätigkeit dieser Pumpe kann für den oben beschriebenen Zweck genutzt werden, indem man eine Leitungsverbindung zwischen dem oberen Teil der venösen Tropfkammer und dem genannten Teilabschnitt herstellt.

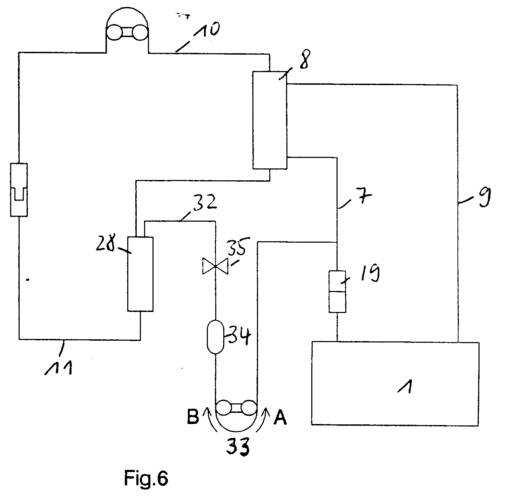

Das Ausführungsbeispiel nach Fig. 6 knüpft an das vorhergehende Beispiel an, bietet aber zusätzliche Funktionen. Der obere Teil der venösen Tropfkammer 28 steht über eine Leitung 32, in die eine Schlauchpumpe 33 eingefügt ist, mit dem Dialysierflüssigkeitssystem in Verbindung. In den Leitungsabschnitt zwischen der venösen Tropfkammer 28 und dieser Pumpe 33 ist außerdem ein Puffergefäß 34 eingefügt, z.B. in Form eines elastischen Beutels mit einem Volumen von typischerweise etwa 20-50 ml. Außerdem ist in den Leitungsabschnitt zwischen der Tropfkammer und dem Puffergefäß 34 ein Ventil 35 eingefügt, vorzugsweise in Form eines Schlauchklemmventils.

Zum Füllen und Spülen des extrakorporalen Blutkreislaufs sind die patientenseitigen Anschlüsse des arteriellen und des venösen Blutschlauches miteinander verbunden. Das Ventil 35 ist geöffnet. Die Pumpe 33 fördert in Richtung A. Dadurch entsteht wie bei dem vorhergehenden Beispiel im Blutschlauchsystem ein Unterdruck, so daß filtrierte Dialysierflüssigkeit durch die Membran des Dialysators 8 in das Blutkompartiment übertritt. Die abgeförderte Luft und die überschüssige Flüssigkeit gelangen über die Pumpe 33 in das Dialysierflüssigkeitssystem.

Nach dem Anschließen des Patienten und dem Beginn der eigentlichen Behandlung dient die in Fig. 6 gezeigte Anordnung dazu, die Dialysemembran entgegen der normalen Filtrationsrichtung periodisch freizuspülen, d.h. Ablagerungen, die sich auf der Blutseite der Membran angesammelt haben, zu entfernen. Es ist bekannt, daß solche Ablagerungen, die eine sogenannte Sekundarmembran bilden und aus Substanzen bestehen, die die Membran nicht passieren können, den effektiven Diffusionswiderstand der Membran vergrößern und dadurch die Effektivität der Blutreinigung, insbesondere hinsichtlich der Ausscheidung höhermoleklarer Schadstoffe, vermindern. Im folgenden wird vorausgesetzt, daß das Dialysierflüssigkeitssystem sich volumenstarr verhält, wie es bei Dialysierflüssigkeitssystemen mit automatischer Bilanzierung (z.B. nach DE-OS 28 38 414) der Fall ist. Das Ventil 35 ist ständig geschlossen. Zum Freispülen der Dialysemembran läuft die Pumpe 33 kurze Zeit (einige Sekunden) mit relativ hoher Fördergeschwindigkeit (in der Größenordnung von 2-10 ml/s) in Richtung A und transportiert Flüssigkeit aus dem Puffergefäß 34 in den Dialysierflüssigkeitskreislauf. Aufgrund des volumenstarren Verhaltens des Dialysierflüssigkeitssystems steigt der Druck im Dialysatkompartiment des Dialysators über den Druck im Blutkompartiment an, so daß eine entsprechende Flüssigkeitsmenge entgegen der normalen Filtrationsrichtung in das Blut übertritt und die aufgelagerten Schichten abschwemmt. In der darauffolgenden Betriebsphase mit normaler Filtrationsrichtung fördert die Pumpe 33 langsam in Richtung B und füllt damit das Puffergefäß 34 wieder auf. Nach einer gewissen Zeit wird die Pumpe wieder in Richtung A in Tätigkeit gesetzt, um das nächste Freispülen der Dialysatormembran einzuleiten. Die Häufigkeit des Freispülens der Dialysatormembran kann nach Bedarf verändert werden. Die Gesamtfiltratbilanz wird von den beschriebenen Vorgängen übrigens nicht beeinflußt, sondern wird, entsprechend der Funktionsweise eines bilanzierenden Dialysierflüssigkeitssystems, von der eingestellten Ultrafiltrationsrate bestimmt.

Die in Fig. 6 gezeigte Anordnung kann darüberhinaus dazu genutzt werden, die Hämodialyse zu einer Hämodiafiltration zu erweitern, d.h. eine Hämodialyse mit wesentlich erhöhter Ultrafiltrationsrate durchzuführen bei gleichzeitiger Substitution des größten Teils der über den Dialysator entzogenen Flüssigkeitsmenge durch Zufuhr einer entsprechenden Menge einer physiologisch angepaßten Lösung in den Blutkreislauf. Bei entsprechender Wahl der Zusammensetzung und gewährleisteter Sterilität der Dialysierflüssigkeit kann diese selbst als Substitutionsflüssigkeit dienen. Zu dem genannten Zweck wird das Ventil 35 geöffnet und die Pumpe 33 mit der Förderrichtung B betrieben. Die Fördergeschwindigkeit wird typischerweise auf 1/3 - 1/5 der Fördergeschwindigkeit der Blutpumpe 4 eingestellt. Unter der Voraussetzung, daß sich das Dialysierflüssigkeitssystem volumenstarr verhält (automatisch bilanzierendes System) entsteht durch die Tätigkeit der Pumpe 33 im Dialysatkompartiment des Dialysators ein zusätzlicher Unterdruck, der eine der Fördergeschwindigkeit der Pumpe 33 entsprechende Filtratmenge aus dem Blutkompartiment in das Dialysatkompartiment übergehen läßt. Die Gesamt-Filtratbilanz wird auch in diesem Falle von den beschriebenen Vorgängen nicht beeinflußt, sondern wird, entsprechend der Funktionsweise eines bilanzierenden Dialysierflüssigkeitssystems, von der eingestellten Ultrafiltrationsrate bestimmt.

Die Anordnung gemäß Fig. 6 kann auch zum Desinfizieren und Spülen des Dialysators verwendet werden. Ein solcher Reinigungszyklus hat zur Folge, daß der Dialysator zur Wiederverwendung aufbereitet werden kann. Dies ist ein weiterer wesentlicher Gesichtspunkt der vorliegenden Erfindung.

Die beschriebenen Funktionen können miteinander kombiniert werden. Eine besonders sinnvolle Kombination ist die Anwendung des periodischen Freispülens der Dialysatormembran bei der Hämodiafiltration, die nach dem beschriebenen oder einem anderen Verfahren durchgeführt wird, weil gerade bei den hohen Filtrationsgeschwindigkeiten, die bei der Hämodiafiltration zur Anwendung gelangen, die Bildung einer den Stoffaustausch hemmenden Sekundärmembran besonders ausgeprägt ist.

Auch andere Teilaspekte der beschriebenen Beispiele können, für den Fachmann leicht ersichtlich, sinnvoll kombiniert werden und durch andere, an sich bekannte Maßnahmen ergänzt werden.

## Patentansprüche

1. Verfahren zum On-Line Spülen und Befüllen eines extrakorporalen Blutkreislaufs von Dialysemaschinen, insbesondere von Blutschlauchleitungen und Dialysatoren, wobei von der Dialysemaschine erzeugte bzw. aufbereitete Dialysierflüssigkeit durch den bzw. in den extrakorporalen Blutkreislauf gefördert wird,
**dadurch gekennzeichnet**,
daß die Dialysierflüssigkeit die Membran des Dialysators passiert und dadurch filtriert wird.

2. Verfahren nach Anspruch 1,
dadurch gekennzeichnet, daß die Dialysierflüssigkeit von der maschineneigenen Blutpumpe durch den bzw. in den extrakorporalen Blutkreislauf gepumpt wird.

3. Verfahren nach Anspruch 1,
dadurch gekennzeichnet, daß die Dialysierflüssigkeit von der Pumpe des Dialysierflüssigkeitskreislaufs durch den bzw. in den extrakorporalen Blutkreislauf gepumpt wird.

4. Verfahren nach Anspruch 1 oder 3,
dadurch gekennzeichnet, daß das Befüllen und Spülen des extrakorporalen Blutkreislaufs durch das maschinenspezifische Füllprogramm hervorgerufen wird.

5. Verfahren nach Anspruch 1 oder 2,
gekennzeichnet durch folgende Schritte:
a) Konnektieren der arteriellen und venösen Blutschlauchleitungen am Dialysator,
b) Verbinden des arteriellen und des venösen Patientenanschlusses der Blutschlauchleitungen zur Schaffung einer durchgehenden Flüssigkeitsleitung,
c) Sperren des Strömungsweges zwischen dem venösen Blutanschluß des Dialysators und der venösen Tropfkammer,
d) Betätigung der Blutpumpe entgegen ihrer normalen Förderrichtung, und
e) Ableiten der aus dem Blutschlauchsystem verdrängten Luft und überschüssigen Flüssigkeit durch eine mit dem oberen Teil der venösen Blutkammer verbundene Leitung.

6. Verfahren nach Anspruch 5,
dadurch gekennzeichnet, daß die verdrängte Luft und die überschüssige Flüssigkeit in das Dialysierflüssigkeitssystem des Dialysegerätes abgeleitet werden.

7. Verfahren nach Anspruch 5 oder 6,
dadurch gekennzeichnet, daß intermittierend die normalen Strömungsverhältnisse des extrakorporalen Blutkreislaufs durch folgende Schritte hergestellt werden:
c) Öffnen des Strömungsweges zwischen dem venösen Blutanschluß des Dialysators und der venösen Tropfkammer,
d) Betätigen der Blutpumpe in ihrer normalen Förderrichtung und
e) Verschließen der mit dem oberen Teil der venösen Tropfkammer verbundenen Leitung.

8. Verfahren nach Anspruch 1 oder 2,
gekennzeichnet durch die Kombination folgender Schritte:
a) Konnektieren der arteriellen und venösen Blutschlauchleitungen am Dialysator,
b) Verbinden des arteriellen und des venösen Patientenanschlusses der Blutschlauchleitung zur Schaffung einer durchgehenden Flüssigkeitsleitung, und
c) Verbinden einer an den oberen Teil der venösen Tropfkammer angeschlossenen Leitung mit einer Saugvorrichtung.

9. Verfahren nach Anspruch 8,
dadurch gekennzeichnet, daß als Saugvorrichtung das Entgasungssystem des Dialysegerätes benutzt wird.

10. Verfahren nach Anspruch 8,
dadurch gekennzeichnet, daß die Saugvorrichtung eine Pumpe (339 ist, deren abfördernde Leitung mit dem Dialysierflüssigkeitssystem verbunden ist.

11. Verfahren nach einem der Ansprüche 1 bis 10,
dadurch gekennzeichnet, daß im Dialysatorkompartiment des Dialysators in bestimmten Zeitabständen ein erhöhter Druck erzeugt wird, der den Druck im Blutkompartiment des Dialysators übersteigt und eine Filtration von Dialysierflüssigkeit in das Blut bewirkt.

12. Verfahren nach Anspruch 11, wobei das Dialysierflüssigkeitssystem volumenstarr wirkt,
dadurch gekennzeichnet, daß eine periodische Druckerhöhung und Druckabsenkung mit der Pumpe (33) bewirkt wird, die abwechselnd zusätzliche Flüssigkeit in das Dialysierflüssigkeitssystem einführt bzw. dem Dialysierflüssigkeitssystem Flüssigkeit entzieht.

13. Verfahren nach einem der Ansprüche 1 bis 12,
dadurch gekennzeichnet, daß die Dialysierflüssigkeit stromaufwärts des Dialysators einen Sterilfilter passiert.

## Claims

1. Process for the on-line flushing and filling of an extracorporeal blood circuit of dialysis machines, in particular of blood tube lines and dialysers, wherein dialysing fluid produced or prepared by the dialysis machine is conveyed through or into the extracorporeal blood circuit, characterised in that the dialysing fluid is passed through the membrane of the dialyser and thereby filtered.

2. Process according to claim 1, characterised in that the dialysing fluid is pumped through or into the extracorporeal blood circuit by the blood pump belonging to the machine.

3. Process according to claim 1, characterised in that the dialysing fluid is pumped through or into the extracorporeal blood circuit by the pump of the dialysing fluid circuit.

4. Process according to claim 1 or 3, characterised in that the filling and flushing of the extracorporeal blood circuit is brought about by the machine-specific filling programme.

5. Process according to claim 1 or 2, characterised by the following steps:
a) connecting the arterial and venous blood tube lines to the dialyser,
b) joining the arterial and the venous patient connection of the blood tube lines to create a continuous fluid line,
c) blocking the flow path between the venous blood connection of the dialyser and the venous drip chamber,
d) operating the blood pump in the direction opposite to its normal direction of delivery, and
e) drawing off the air displaced from the blood tube system and excess fluid through a line which is joined to the upper part of the venous blood chamber.

6. Process according to claim 5, characterised in that the displaced air and the excess fluid are drawn off into the dialysing fluid system of the dialysis apparatus.

7. Process according to claim 5 or 6, characterised in that the normal flow conditions of the extracorporeal blood circuit are produced intermittently by the following steps:
c) opening the flow path between the venous blood connection of the dialyser and the venous drip chamber,
d) operating the blood pump in its normal direction of delivery and
e) closing the line joined to the upper part of the venous drip chamber.

8. Process according to claim 1 or 2, characterised by the combination of the following steps:
a) connecting the arterial and venous blood tube lines to the dialyser,
b) joining the arterial and the venous patient connection of the blood tube line to create a continuous fluid line, and
c) joining a line connected to the upper part of the venous drip chamber to a suction device.

9. Process according to claim 8, characterised in that the degassing system of the dialysis apparatus is used as the suction device.

10. Process according to claim 8, characterised in that the suction device is a pump (33), the delivering line of which is joined to the dialysing fluid system.

11. Process according to one of claims 1 to 10,
characterised in that an elevated pressure which exceeds the pressure in the blood compartment of the dialyser and causes dialysing fluid to filter into the blood is produced at certain intervals in the dialyser compartment of the dialyser.

12. Process according to claim 11, wherein the dialysing fluid system operates with a fixed volume, characterised in that a periodic pressure increase and pressure decrease is effected by the pump (33), which alternately introduces additional fluid into the dialysing fluid system and removes fluid from the dialysing fluid system.

13. Process according to one of claims 1 to 12,
characterised in that the dialysing fluid passes through a sterile filter upstream of the dialyser.

## Revendications

1. Procédé de nettoyage et de remplissage en ligne d'un circuit extracorporel sanguin de machines de dialyse, notamment de conduits sanguins en tuyau souple et de dialyseurs, où le liquide de dialyse produit ou préparé par la machine de dialyse est convoyé à travers respectivement dans le circuit extracorporel sanguin, caractérisé en ce que le liquide de dialyse passe à travers la membrane du dialyseur et est ainsi filtré.

2. Procédé selon la revendication 1,
caractérisé en ce que le liquide de dialyse est pompé par la pompe de sang propre à la machine à travers respectivement dans le circuit extracorporel sanguin.

3. Procédé selon la revendication 1,
caractérisé en ce que le liquide de dialyse est pompé par la pompe du circuit de liquide de dialyse à travers respectivement dans le circuit extracorporel sanguin.

4. Procédé selon la revendication 1 ou 3,
caractérisé en ce que le remplissage et le nettoyage du circuit extracorporel sanguin sont provoqués par le programme de remplissage spécifique à la machine.

5. Procédé selon la revendication 1 ou 2,
caractérisé par les étapes suivantes :
a) connexion des conduits sanguins artériels et veineux au dialyseur,
b) liaison du raccordement artériel et veineux du patient des conduits sanguins pour créer un conduit de liquide continu,
c) blocage du trajet d'écoulement entre le raccordement de sang veineux du dialyseur et la chambre de goutte-à-goutte veineuse,
d) actionnement de la pompe de sang contre sa direction de convoyage normale,
e) évacuation de l'air refoulé du système de conduit sanguin et du liquide excédentaire par un conduit relié à la partie supérieure de la chambre de sang veineux.

6. Procédé selon la revendication 5,
caractérisé en ce que l'air refoulé et le liquide excédentaire sont évacués dans le système de liquide de dialyse de l'appareil de dialyse.

7. Procédé selon la revendication 5 ou 6,
caractérisé en ce que par intermittence, les conditions de flux normal du circuit extracorporel sanguin sont réalisées par les étapes suivantes :
c) ouverture du trajet d'écoulement entre le raccordement de sang veineux du dialyseur et de la chambre de goutte-à-goutte veineuse,
d) actionnement de la pompe de sang dans la direction de convoyage normale et
e) fermeture du conduit relié à la partie supérieure de la chambre de goutte-à-goutte veineuse.

8. Procédé selon la revendication 1 ou 2, caractérisé par la combinaison des étapes suivantes :
a) connexion des conduits de sang artériel et veineux au dialyseur,
b) liaison du raccordement artériel et veineux du patient du conduit sanguin pour créer un conduit de liquide continu et
c) liaison d'un conduit raccordé à la partie supérieure de la chambre de goutte-à-goutte veineuse à un dispositif d'aspiration.

9. Procédé selon la revendication 8,
caractérisé en ce qu'il est utilisé comme dispositif d'aspiration le système de dégazage de l'appareil de dialyse.

10. Procédé selon la revendication 8,
caractérisé en ce que le dispositif d'aspiration est une pompe (339) dont le conduit d'évacuation est relié au système du liquide de dialyse.

11. Procédé selon l'une des revendications 1 à 10,
caractérisé en ce qu'il est produit dans le compartiment du dialyseur à des intervalles de temps déterminés une plus grande pression qui dépasse la pression dans le compartiment sanguin du dialyseur et qui provoque une filtration du liquide de dialyse dans le sang.

12. Procédé selon la revendication 11, où le système du liquide de dialyse a un effet de rigidité volumique,
caractérisé en ce qu'il est provoqué une augmentation et une diminution périodiques de la pression au moyen de la pompe (33) qui introduit alternativement du liquide additionnel dans le système de liquide de dialyse et qui retire respectivement du liquide du système de liquide de dialyse.

13. Procédé selon l'une des revendications 1 à 12,
caractérisé en ce que le liquide de dialyse passe à travers un filtre stérile en amont du dialyseur.
